# EUROPEAN PATENT SPECIFICATION

(11) **EP 3 308 757 B1**
(45) Date of publication and mention of the grant of the patent: **17.11.2021**
(21) Application number: 17196000.8
(22) Date of filing: 11.10.2017
(51) Int. Cl.: A61F 9/00

(54) **DEVICE FOR OCULAR ADMINISTRATION OF FLUIDS**
VORRICHTUNG ZUR AUGENVERABREICHUNG VON FLUIDEN
DISPOSITIF POUR ADMINISTRATION OCULAIRE DE FLUIDES

(30) Priority: 12.10.2016 IT 201600102531
(43) Date of publication of application: 18.04.2018
(73) Proprietor: Università degli Studi di Parma, 43121 Parma (IT)
(72) Inventor: RONDA, Nicoletta, 43124 Parma (IT); BETTINI, Ruggero, 43124 Parma (IT); BERNINI, Franco, 43124 Parma (IT)
(74) Representative: Gerli, Paolo

(56) References cited:
- CA-A1- 2 027 632
- CA-A1- 2 177 398
- FR-A1- 2 368 936
- FR-A1- 2 717 680
- US-A- 3 016 898
- US-A- 4 468 103
- US-A- 4 733 802
- US-A1- 2013 245 579
- elliot bray: "Ezy Drop Guide and Eye Wash Cup", youtube, 22 September 2016 (2016-09-22), page 1 pp., XP054977589, Retrieved from the Internet: URL:https://www.youtube.com/watch?v=pAkr9z 8Gqkc [retrieved on 2017-07-25]

## Description

### FIELD OF THE INVENTION

The invention consists of a device for directing and controlling the ocular administration of a fluid, suitable in particular, but not exclusively, for self-administration.

### PRIOR ART

The self-administration of eye drops or other fluids intended for the eyes is a procedure which is apparently simple, but in fact poses a number of risks and is not easy, in particular for elderly persons or people who have difficulty performing delicate manual movements.

Risks: the dispensing bottle must not come into contact with the skin or with the eye structures in order to avoid microbiological contamination of the product or damage and/or conjunctival and corneal infections. The procedure commonly used of lowering the bottom eyelid using the free hand in order to create a zone for receiving the drops, with the risk of compression, traction or dislocation of the eyeball, could be harmful in the case where there have been recent eye operations or traumas, i.e. conditions for which the frequent administration of eye drops is indeed recommended.

Difficulties with performing the maneuver: the need to use the force of gravity in order to cause the eye drops to fall into the conjunctival sac makes the successful outcome of the operation dependent on the correct relative positioning of the dispenser and the ideal plane passing along the surface of the eye. This operation is particularly critical in view of the small dimensions of the opening of the palpebral fissure. Finally, particular difficulties in coordinating and controlling the delicate movements of the hands in given persons, such as the elderly, make this operation very problematic, if not impossible. Finally, the frequent need to repeat the attempts at administering the eye drops also results in the wastage of product.

Different devices able to facilitate the self-administration of eye drops are known: they consist in general of devices of various shapes and sizes which can be positioned on the eye and which comprise an eye drop dispenser which is fixed at a suitable distance from the contact surface of the eye: once the device has been positioned on the eye it is sufficient to perform dispensing of the liquid from the dispenser: the eye drops fall onto the surface of the eye without the user having to worry about orienting or manually positioning the dispenser with respect to the eye or preparing the eye for administration.

For example, US patent application 2015/0351960 describes an eye drop dispenser provided with a positioning member which allows the dispenser to be aligned with the eye; the positioning member is formed by two spacer arms, the proximal ends of which are fixed to the dispenser and the distal ends of which are joined together by a crosspiece (bridge piece) with a curved shape designed to surround part of the eye socket; when the surface of the bridge piece is rested on the contour of the eye the dispenser is positioned suitably spaced from the eye socket; when the dispenser is operated the drops fall onto the eye; however, the device does not ensure precise and stable positioning on the eye; in fact, once rested on the eye socket, it is freely inclinable so that, during dispensing, the nozzle of the dispenser may be located in a non-optimal position relative to the eye and irrigate it peripherally or even externally.

Other devices attempt to ensure correct positioning on the eye by means of fixing on the nose: for example, the application WO2014/205556 illustrates a nasal locator, which is similar to a bridge piece of eyeglasses and can be applied to the nose, having extending from it in opposite directions two arms directed towards the right eye and left eye; a hopper, which has its spout positioned above the eye, is mounted on each arm, at the height of the eye zone, at a suitable distance from the surface of the eye; during application the user dispenses the eye drops into the hopper from a dispenser held in one hand. The device has the drawback that the liquid flows down the walls of the hopper with the risk of being contaminated and requiring washing of the device after each use; moreover, the bridge piece may be accidentally moved forwards along the longitudinal axis of the nose, resulting in the hoppers being out of alignment with the eye and/or the risk of the dispenser nozzle being lowered towards the corneal surface. The same drawbacks are present in the device illustrated in US 2015/0088099 which also makes use of a fixing nose piece in order to position the dispenser over the eye zone.

WO2015/123656 describes a device in the form of eyeglasses which, in the zone normally occupied by the lenses, incorporates liquid dispensers and a complex computerized system of sensors and microcameras which allows optimization of the duration and the directionality of application of the liquid; however, the complexity and the cost of such a device are such that the product has a limited applicability in real terms.

US2015/0164688 describes a spacer with a frustoconical form, the top opening of which surrounds the eye and the bottom opening of which receives the nozzle of a dispenser; the shape of the device and the positioning of the bottom opening are chosen so as to leave free an internal space which houses a LED and associated battery: at the moment of administration the device is rested on the eye socket and the LED is illuminated: the light emitted stimulates the patient to open the eye during administration.

CA2,027,632 discloses an eye dropper with the shape of a truncated cone, with a contoured base and a wall extending therefrom to an aperture, which is of a size to receive a nozzle of an eye drop container; based on the approximate estimation that the human orbital margin is more or less invariant in humans, the device is in "one-size-fits-all", applicable to both left and right eye interchangeably; although the contoured base is said to fit to the orbital margin of the human orbit, the device is not adapted to fit closely to both human orbits, which normally present different depths in their upper and lower orbital arches; the resulting approximate fitting to both orbits translates in an approximate positioning of the device with respect to the centers of the eyes upon which the liquid is to be dropped.

All the solutions which are currently available, although useful for facilitating self-administration of liquids into the eye, have limitations as regards one or more of the following characteristics: stability of the dispenser, spacing from the corneal surface and/or precise guiding of the drops onto the eye; the latter aspect in particular has not been fully investigated and the current devices have obvious limitations in terms of precision.

### SUMMARY

A new device for facilitating self-administration of a fluid into a patient's conjunctival sac has now been developed by the Applicant, said device advantageously combining in a single product optimum characteristics in terms of stability, positioning, spacing of the fluid dispenser and guiding of the dispensed fluid - without any contact with the device - into the center of the eye, with the further advantage of a high structural simplicity and low constructional cost.

The device has the shape of a hollow truncated cone (1), the minor (top) base (2) of which is adapted to house the dripper of a fluid dispenser and the major (bottom) base (3) of which has a non-coplanar ovoid shape adapted to rest precisely on the periocular area of the patient, i.e. so as to surround it and match its form. The device allows, during application to the eye, safe spacing of the tip of the dripper (housed on the minor base) from the corneal surface, preventing accidental contact between dripper and cornea.

According to the present invention, all the aforementioned stability, positioning, spacing and guiding characteristics are determined or at least dependent on the perimeter of the major base (3) of the device which has been designed not only to surround, but also to match as closely as possible the form of the periocular area; the device rests precisely, in a unique position and stably around the natural form of the eye, eliminating or reducing as far as possible the possibility of movements of the device and/or misalignment with respect to the eye socket. The positioning stability/precision of the device thus obtained is such that a dripper housing point (5) may be precisely provided on the minor base of the device so that, during application to the eye, it is located vertically aligned with the center of the eye. This point, owing to the non-symmetrical structure of the periocular area, does not coincide with the geometrical axis of the frustoconical device, but is laterally displaced. Owing to the aforementioned arrangement of the parts, during use with the patient lying in a supine position, the drop released by the dripper, is guided by the force of gravity directly into the center of the eye.

In order to obtain the aforementioned results, the combination of two structural characteristics is particularly important: the non-planarity of the perimeter of the major base (3) (which follows the natural undulation of the periocular area, with the provision of suitable concavities and convexities) and the lateral displacement of the housing for the dripper on the top base (chosen so that it is situated vertically aligned with the center of the eye during use).

### DESCRIPTION OF THE FIGURES

Figure 1: administration device according to the invention, in the form of one article for application either to the right or left eye. Figure 1a: perspective view; Figure 1b: front view; Figure 1c: side view; Figure Id: cross-sectioned side view; Figure 1e: cross-sectioned top plan view.
Figure 2: administration device according to the invention, in the form of an article for application to the left eye. Figure 2a: perspective view; Figure 2b: front view; Figure 2c: side view.
Figure 3: administration device according to the invention, in the form of an article for application to the right eye. Figure 3a: perspective view; Figure 3b: front view; Figure 3c: side view.
Figure 4: schematic representation of the center of the eye.

### DESCRIPTION OF THE INVENTION

The present invention relates to a device called an Eye Drop Aid (EDA) (1) for administering a fluid to the eye of a patient, having the shape of a hollow truncated cone, with a minor base (2) and a major base (3) where the major (bottom) base (3) has the shape of an ovoid and has a non-coplanar perimeter suitable for surrounding the periocular area of the patient and for matching its form; the minor (top) base (2) has a housing (4) for the dripper of a liquid dispenser, said housing having the shape of an inverted truncated cone with a hole (5) and being positioned so that, upon application of the major base (3) onto the eye, the hole lies vertically aligned with the center of the eye. During application, the tip of the dripper housed on the top minor base (2) remains spaced from the corneal surface, and the fluid administered from the dripper does not come into contact with the device. As further described, the device allows the distal part of the dripper (i.e. the part containing the dripper tip) to protrude into the internal of the device for a length sufficient to allow dripping without the fluid contacting the device and, at the same time, without the dripper contacting the corneal surface.

In the present text, the terms "top" and "bottom" indicate the orientation of the frustoconical bases of the device during application to the eye of the patient lying in a supine position, where the minor base is arranged at the top and the major base is arranged at the bottom.

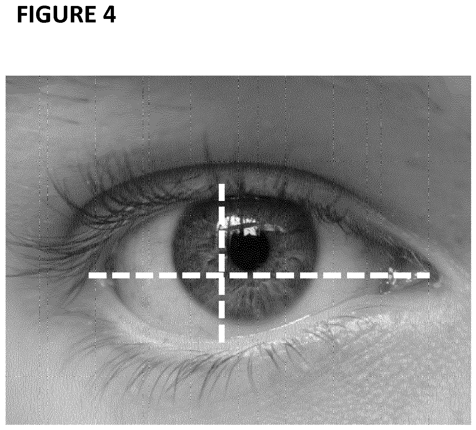

In the present text "center of the eye" is understood as meaning the point of intersection between the horizontal line which connects the lateral ends of the eye and the vertical line which defines the maximum amplitude of the palpebral fissure (Figure 4).

In the present text "maximum diameter" of the ovoid is understood as meaning the diameter of the ovoid measured at its point of maximum extension. In the present text "minimum diameter" of the ovoid is understood as meaning the diameter of the ovoid measured at its point of minimum extension.

In the present text "positioning point" of the dripper is understood as meaning the center (hole) of the area intended to house the dripper; accordingly, the terms "positioning point of the dripper" and "hole" are synonymous in the present text and can be used interchangeably depending on the context; moreover, when coordinates (i.e. distances) are given to localize said positioning point/hole, they are are meant to be measured with respect to the geometric center of said hole.

The expression "the fluid administered from the dripper does not come into contact with the device" used herein means that, on application, the liquid delivered from the tip of the dripper falls directly onto the corneal surface without intercepting any parts of the present device (excluding of course the dripper and the fluid dispenser themselves)

An important characteristic feature of the invention is the high precision with which the bottom major base (3) (i.e. its perimeter) matches the anatomy of the periocular area: it has the shape of an ovoid which is suitable for surrounding the eye and is also non-coplanar, namely comprises concavities (6) and convexities (7) matching those naturally present in the periocular area; more specifically, the ovoidal perimeter is concave in the two median portions (6) of the ovoid and convex in the two elongated parts (7). The aforementioned shape characteristics are understood as having dimensions on the scale of the human eye. In particular the bottom base (3) has a maximum diameter (8) of the ovoid preferably comprised between 38 mm and 49 mm, more preferably between 41 mm and 46 mm and a minimum diameter of the ovoid (9) preferably comprised between 26 mm and 34 mm, more preferably between 28 mm and 32 mm.

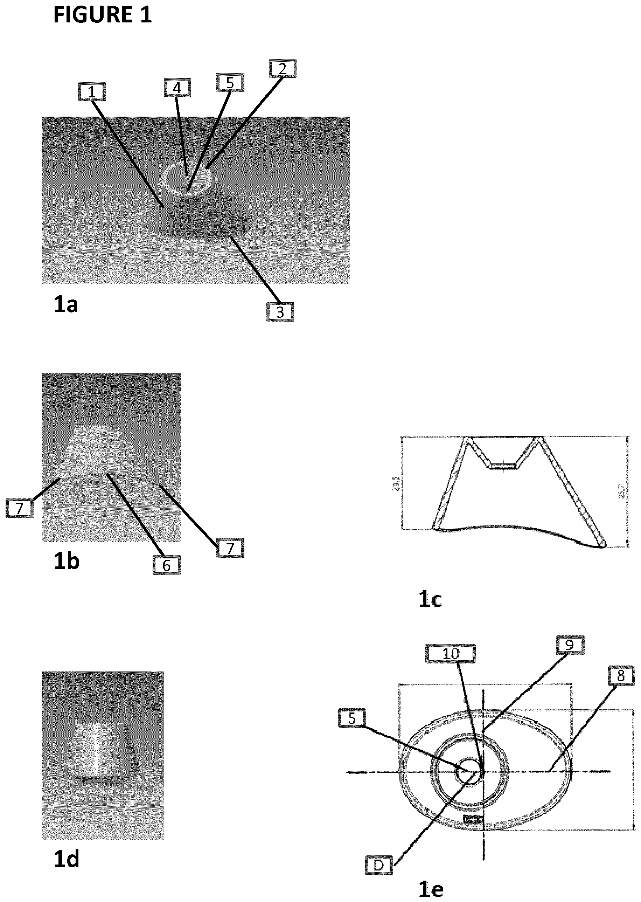

As previously indicated, the minor base (2) has a housing (4) having the shape of an inverted truncated cone (where the term "inverted" is understood as relative to the orientation of the truncated cone which forms the device), with a hole (5) in the apex of the cone (Figure 1a). The diameter of the hole is such as to allow the dripper, i.e. a portion thereof, but not the dispenser, to pass through it; the dripper has a tubular cross-section which is variable, namely decreasing (tapered) in the direction of the tip: the hole (5) allows the tip of the dripper to pass through, stopping it at a safe distance from the surface of the eye when the maximum diameter of the dripper, measured at in correspondence of the hole, is greater than the diameter of the hole. A typical range of dimensions for the hole diameter is comprised between 4 mm and 7 mm; different values are possible, being chosen always in accordance with the criterion mentioned above, depending on the shape and size of the particular dripper being used. In an embodiment, the device is supplied jointly with a liquid dispenser comprising a dripper, where the dripper specifically matches the chosen diameter of the hole (5); the term "specifically matches" means that the distal part of the dripper (i.e. the one containing the tip), measured from the level where its tapered cross-section equals the diameter of the hole, to the end of the tip, has a length comprised between 3 and 5 mm; this matching ensures that, after positioning and stopping of the dripper into the hole/positioning point (5), the dripper tip remains safely distanced from the corneal surface: this allows dripping without the fluid contacting the device and, at the same time, without the dripper contacting the corneal surface.

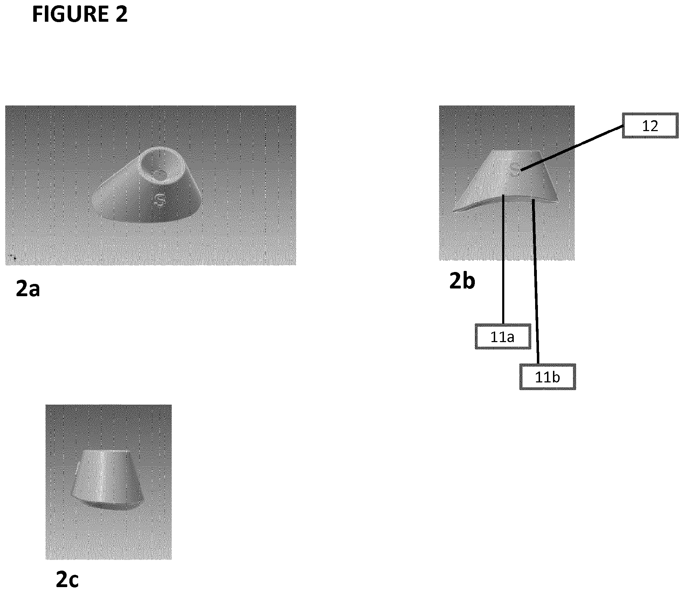

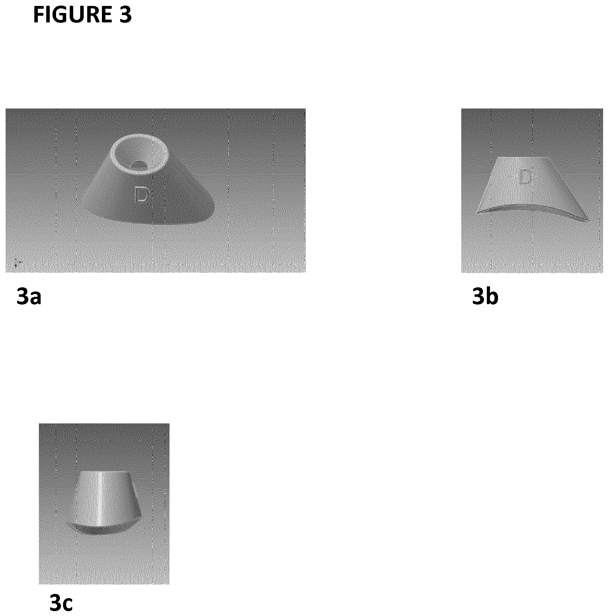

As previously indicated, the bottom base (3) of the present device not only "surrounds" the eye, but also matches as closely as possible the profile of the periocular area. Consequently the device may be easily applied by the patient (or an assistant) in substantially univocal position relative to the eye to be treated: in fact a position significantly different from the anatomically complementary position generates "signals" which the patient is able to perceive: for example an excessive pressure of the device on certain zones of the ocular perimeter, or the instability of the device, or the perception of zones which do not adhere properly, indicated by the infiltration of light from outside. The unique positioning relative to the eye has allowed the inventor to identify precisely, during development of the invention, the point (5) of the top base (2) of the device which, during application, is located perpendicular to the center of the eye: the zone for housing the dripper has been provided at this point. In order to obtain this result, the point where the dripper is positioned on the top base is situated along (i.e. it lies vertically on) the maximum diameter (8) of the bottom ovoidal base, at a distance (D) from the center (10) of the ovoid (measured on the diameter) comprised between 2 mm and 6 mm (Figure 1e). The point (5) where the dripper is positioned is therefore displaced along the maximum diameter, relative to the geometric center (10) of the ovoid. The resultant asymmetrical configuration of the device is suitable for the right eye or left eye, depending on the direction of displacement (right or left) of the positioning point of the dripper. However, the same device, rotated through 180°, assumes the opposite configuration: therefore it may be supplied to the patient as a single article (Figure 1), which can be rotationally oriented depending on the eye to be treated; alternatively, in a preferred embodiment, it may be supplied as two opposite asymmetrical (i.e. specular) articles (Figures 2, 3), preferably marked as "right" and "left" or the like (e.g. D / S in Figures 2, 3). This variant allows an even more precise adaptation of the device to the anatomy of the periocular area, by providing the two median concavities (11a, 11b) of the bottom major base with different depths, being complementary to the corresponding convexities present in the periocular area, in particular the slightly more projecting profile of the superciliary arch compared to that of the bottom arch of the orbit; the adherence/stability of the device in the periocular area is thus further increased.

The distance between the bottom base (3) and the end of the inverted truncated cone with the hole (corresponding to the point (5) where the dripper is positioned) is chosen as to ensure, during application, a sufficient spacing between the tip of the dripper and the surface of the patient's eye; typically, this spacing is equal to about 12 mm; preferred reference values are comprised between 10 mm and 15 mm.

The inverted frustoconical shape of the housing (4) for the dripper allows the dripper to be stably fixed at the positioning point (5), also allowing the dispensed fluid to fall onto the eye without coming into contact with the device, i.e. touching the walls of the device. Said "stable fixing" of the dripper may be performed temporarily, semi-permanently or permanently; temporary fixing may be performed by inserting the tip of the dripper inside the hole (i.e. by partially passing the dripper through the hole until halt), to be carried out at the moment of administration; semi-permanent fixing may be performed by providing engaging and disengaging means for the dripper in the region of its positioning point (5) on the top base, for example a threaded hole inside which the dripper may be screwed/unscrewed; permanent fixing may be performed by connecting the dripper to its positioning point on the top base in an irreversible manner: in this case, the dripper and, where present, the dispenser connected to it form an integral and fixed part of the device according to the invention.

Owing to the aforementioned constructive characteristics, the dripper housed on the device dispenses the fluid through the said device without the fluid coming into contact with the device, namely without it touching its walls.

In accordance with the present invention, the term "dripper" is understood so as to include any structure through which it is possible to dispense a fluid in small amounts suitable for ocular administration, not necessarily in the form of drops. The dripper moreover is not limited to any specific constructional form and includes any structure through which small volumes of fluids, drops, droplets, atomized droplets or gases may be dispensed. The fluid may be any fluid intended for ocular administration, e.g. a liquid, a gel or a paste, preferably a liquid. From a medical point of view it may consist of eye drops, an eyewash or any other type of medication for the eyes.

The administration device according to the invention may be made of different materials which are compatible with pharmaceutical use and in particular suitable for contact with the eye and the periocular areas. A preferred material is polypropylene for alimentary use, which is biologically inert and anti-bacterial, non-toxic, very resistant to the action of salts, acids, alkalis, alcohol and detergents and is entirely recyclable. Polypropylene moreover has a pleasant feel and does not produce cutting edges when broken. It is opaque, but semi-transparent, this being a characteristic useful for the intended use, since the patient retains a certain degree of visibility, while having the sensation that the eye is protected. Optionally, in order to improve further the comfort, the sealing effect and adherence to the face it is possible to add an insert made of soft material, e.g. rubbery material, along the perimeter of the bottom major base.

During administration the device according to the invention is rested with its bottom base on the periocular area of the patient who has previously assumed the supine position; the dripper (if not already housed in the device) is positioned manually in its housing point; then, by means of a suitable action, dependent on the type of dripper/dispenser used, the fluid is dispensed through the device; owing to the aforementioned arrangement of structural elements, the fluid falls by means of the force of gravity close to the center of the eye. During all the stages of administration, the fluid never comes into contact with the device (in this sentence the term "device" does not include the dripper and the dispenser). This construction differs from conventional devices in which the tip of the dripper only rests upon a (perforated) surface of the top base: in this case residues of the delivered fluid may, for surface tension reasons, remain adherent to the base in correspondence of its contact point with the tip of the dripper: this may cause a reduction of the effective administered dose and a contamination of the device itself, which should be avoided, especially in case of re-usable devices.

The invention comprises the administration device described herein as a product as such. A device, which is not part of the invention, may be provided in the form of a single article (Figure 1) applicable to one of the two eyes (or to the other one after rotation of the device through 180°); optionally, the device may contain on the outer surface markings suitable for identifying the correct orientation for application to each eye. The device is provided in the form of two asymmetrical articles intended respectively for the right eye and left eye (Figures 3,2); whereby the outer surface contains markings (12) for identifying the device suitable for each eye. In both cases the device may be provided for single use (disposable device) or for repeated use. The disposable devices may be provided in multi-unit packages.

The invention relates furthermore to a kit comprising the administration device as described hitherto in association with a suitable quantity of fluid (eye drops, eyewash, etc.), which may be administered with it. Said quantity of fluid may be contained inside a dispenser provided with dripper to be applied to the device during administration, in the modes indicated above; alternatively, the dispenser and dripper may be present in the kit as separate components which are assembled together during use. The kit may also comprise an eye protection mask and/or any other complementary object useful for ocular administration.

The use of the device described herein , which is not part of the invention, is for the administration of a fluid pharmaceutical compound (aerosol, eye drops, eyewash, gel, paste, etc.) to the eye of a patient in need thereof.

A method of administering a fluid pharmaceutical compound to an eye of a person in need thereof, which is not part of the invention, uses the device described herein: in particular the method is accomplished by: (a) positioning the major base (3) of the device onto said eye; (b) inserting the dripper tip of the fluid dispenser into the hole (5) until its blockage at safe distance from the eye; (c) dispensing the fluid from the dispenser. The step (b) is avoided when the dripper and dispenser are provided permanently integrated with the device. The step (c) is performed in conventional ways (e.g. by pressure of a deformable reservoir, by pressure on a piston, by actuating a hydraulic system, etc.) depending on the particular structure of chosen dispenser/reservoir.

In particular, during use, the device of the present invention incorporates and performs the functions of a spacer, positioner and stabilizer of the dripper and device for directing the drops into the center of the eye. By means of these functions incorporated in the present device (EDA) it is possible to perform self-administration of the eye drops in a manner which is safe and reassuring for the patient.

The device is preferably designed for use in self-administration mode, thus providing the most advantageous application of the present invention; the invention, however, also envisages use of the device for administration by third parties (e.g. an assistant, family member, etc.), resulting in any case in easier administration compared to the conventional mode.

The administration of the fluid to the center of the eye, realized by the present invention, reduces to a minimum the risk of dispersion of the fluid which is administered. The device may be easily made by means of single-piece molding without need for additional constructional parts and without inserting control/guide instruments such as lights, sensors, microcameras, etc., while nevertheless ensuring precise administration in the center of the eye. Owing to its low production cost the product is also available to the public at a low cost in the form of multiple single-use units or in any case units which can be replaced after a limited number of uses, thus increasing the hygiene and versatility of use of the product.

## Claims

1. A device for administering a fluid to the eye of a patient in the form of two articles, respectively suitable for application to the right or left eye, comprising on its outer surface markings for identifying the article suitable for application to the right or left eye, each article having the
shape of a hollow truncated cone (1), with a minor base (2) and a major base (3), wherein:
(a) the major base (3) has the shape of an ovoid and has a non-coplanar perimeter, suitable for surrounding the periocular area of the patient and for matching its form;
(b) the minor base (2) comprises a housing (4) for a dripper of a liquid
dispenser, said housing (4) having the shape of an inverted truncated cone
with a hole (5) and being positioned such that, upon application of the major base (3) to the eye, the hole (5) lies vertically aligned with the center
of the eye;
(c) such that during application, the tip of the dripper when housed on the minor base
(2) remains spaced from the corneal surface, and the fluid administered from the dripper does not come into contact with the device, whereby the non-coplanar perimeter has, in the two median portions of the ovoid, concavities (11a, 11b) having different depths.

2. Device according to claim 1, wherein said major base (3) having the shape of an ovoid has a maximum diameter (8) comprised between 38 and 49 mm and a minimum diameter (9) comprised between 26 and 34 mm.

3. Device according to any one of claims 1-2, wherein said non-coplanar perimeter is concave in the two median portions (6) and convex in the two elongated parts (7) of the ovoid described by it.

4. Device according to any one of claims 1-3, wherein the hole Z (5) lies
vertically on the maximum diameter of the ovoid of the major base (3), at a
distance from the center of the ovoid comprised between 2 and 6 mm.

5. Device according to any one of claims 1-4, comprising the dripper temporarily, semi-permanently, or permanently housed in the minor base (2).

6. Device according to any one of claims 1-5, comprising the dispenser connected to the dripper.

7. A kit comprising the device described in any one of claims 1-6, in association with a reservoir for the fluid to be administered.

## Patentansprüche

1. Vorrichtung zum Verabreichen eines Fluids an ein Auge eines Patienten in Form zweier Gegenstände, die für die Anwendung auf das rechte bzw. das linke Auge geeignet sind, die auf ihrer äußeren Oberfläche Markierungen aufweisen, um den Gegenstand, der für die Anwendung auf das rechte oder das linke Auge geeignet ist, zu identifizieren, wobei jeder Gegenstand die Form eines hohlen Kegelstumpfs (1) mit einer kleinen Basis (2) und einer großen Basis (3) hat, wobei:
(a) die große Basis (3) die Form eines Ovals besitzt und einen nicht koplanaren Umfang aufweist, der einen nicht periokularen Bereich des Patienten umgeben kann und sich an dessen Form anpassen kann;
(b) die kleine Basis (2) ein Gehäuse (4) für einen Tropfer eines Flüssigkeitsspenders aufweist, wobei das Gehäuse die Form eines umgedrehten Kegelstumpfs mit einem Loch (5) hat und so positioniert ist, dass beim Aufbringen der großen Basis (3) auf das Auge das Loch vertikal ausgerichtet auf das Zentrum des Auges liegt;
(c) so dass während der Anwendung die Spitze des Tropfers, wenn er in der kleinen Basis (2) aufgenommen ist, von der Hornhautoberfläche beabstandet bleibt und das von dem Tropfer verabreichte Fluid nicht mit der Vorrichtung in Kontakt gelangt,
wobei der nicht koplanare Umfang in den beiden Medianabschnitten des Ovals Konkavitäten (11a, 11b) mit unterschiedlichen Tiefen besitzt.

2. Vorrichtung nach Anspruch 1, wobei die große Basis (3), die die Form eines Ovals besitzt, einen maximalen Durchmesser (8) im Bereich von 38 bis 49 mm und einen minimalen Durchmesser (9) im Bereich von 26 bis 34 mm hat.

3. Vorrichtung nach einem der Ansprüche 1-2, wobei der nicht koplanare Umfang in den beiden Medianabschnitten (6) konkav ist und in den beiden länglichen Abschnitten (7) des Ovals, das dadurch beschrieben wird, konvex ist.

4. Vorrichtung nach einem der Ansprüche 1-3, wobei das Loch (5) auf dem maximalen Durchmesser des Ovals der großen Basis (3) vertikal und in einem Abstand vom Zentrum des Ovals, der im Bereich von 2 bis 6 mm liegt, liegt.

5. Vorrichtung nach einem der Ansprüche 1-4, wobei der Tropfer vorübergehend, halb dauerhaft oder dauerhaft in der kleinen Basis (2) aufgenommen ist.

6. Vorrichtung nach einem der Ansprüche 1-5, die den mit dem Tropfer verbundenen Spender aufweist.

7. Baugruppe, die die Vorrichtung nach einem der Ansprüche 1-6, die einem Vorratsbehälter für das zu verabreichende Fluid zugeordnet ist, umfasst.

## Revendications

1. Dispositif pour administrer un fluide dans l'œil d'un patient sous la forme de deux articles, aptes à être appliqués respectivement sur l'œil droit ou l'œil gauche, comprenant sur sa surface extérieure des marques pour identifier l'article apte à être appliqué sur l'œil droit ou l'œil gauche, chaque article ayant la forme d'un tronc de cône creux (1), avec une petite base (2) et une grande base (3), où :
(a) la grande base (3) a la forme d'un ovoïde et a un périmètre non coplanaire, apte à entourer la zone périoculaire du patient et à correspondre à sa forme ;
(b) la petite base (2) comprend un logement (4) pour l'embout compte-gouttes d'un distributeur de liquide, ledit logement (4) ayant la forme d'un tronc de cône inversé avec un trou (5) et étant positionné de telle sorte que, lors de l'application de la grande base (3) sur l'œil, le trou (5) se trouve aligné verticalement avec le centre de l'oeil ;
(c) de telle sorte que pendant l'application, l'extrémité de l'embout compte-gouttes, lorsqu'elle est logée sur la petite base (2), reste espacée de la surface cornéenne, et le fluide administré à partir de l'embout compte-gouttes n'entre pas en contact avec le dispositif, le périmètre non coplanaire présentant, dans les deux parties médianes de l'ovoïde, des concavités (11a, 11b) ayant des profondeurs différentes.

2. Dispositif selon la revendication 1, dans lequel la grande base (3) ayant la forme d'un ovoïde a un diamètre maximal (8) compris entre 38 et 49 mm, et un diamètre minimal (9) compris entre 26 et 34 mm.

3. Dispositif selon l'une quelconque des revendications 1-2, dans lequel le périmètre non coplanaire est concave dans les deux parties médianes (6), et convexe dans les deux parties allongées (7) de l'ovoïde qu'il décrit.

4. Dispositif selon l'une quelconque des revendications 1 à 3, dans lequel le trou (5) se situe verticalement sur le diamètre maximal de l'ovoïde de la grande base (3), à une distance du centre de l'ovoïde comprise entre 2 et 6 mm.

5. Dispositif selon l'une quelconque des revendications 1 à 4, comprenant l'embout compte-gouttes logé de manière temporaire, semi-permanente ou permanente dans la petite base (2).

6. Dispositif selon l'une quelconque des revendications 1 à 5, comprenant le distributeur relié à l'embout compte-gouttes.

7. Kit comprenant le dispositif décrit dans l'une quelconque des revendications 1-6, en association avec un réservoir pour le fluide à administrer.
